(19) Europäisches Patentamt
European Patent Office
Office européen des brevets

(11) Veröffentlichungsnummer: **0 298 405**
**A2**

(12) # EUROPÄISCHE PATENTANMELDUNG

(21) Anmeldenummer: **88110642.1**

(22) Anmeldetag: **04.07.88**

(51) Int. Cl.⁴: **C07D 513/04 , C07D 498/04 , A01N 43/90 , //(C07D513/04, 277:00,209:00),(C07D513/04, 277:00,221:00),(C07D498/04, 263:00,221:00),(C07D498/04, 263:00,209:00),(C07D513/04, 279:00,221:00)**

(30) Priorität: **07.07.87 DE 3722827**

(43) Veröffentlichungstag der Anmeldung:
**11.01.89 Patentblatt 89/02**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Franke, Wilfried, Dr.**
**Spiessergasse 6 B**
**D-1000 Berlin 27(DE)**
Erfinder: **Blume, Friedhelm, Dr.**
**Nusshäherstrasse 47 T**
**D-1000 Berlin 27(DE)**
Erfinder: **Arndt, Friedrich, Dr.**
**Mühlenfeldstrasse 10**
**D-1000 Berlin 28(DE)**
Erfinder: **Rees, Richard, Dr.**
**Speerweg 8**
**D-1000 Berlin 28(DE)**

(54) **Anellierte Imino-azole und Imino-azine, Verfahren zu ihrer Herstellung und ihre Verwendung als Mittel mit herbizider Wirkung.**

(57) Die Erfindung betrifft neue anellierte Imino-azole und Imino-azine der allgemeinen Formel I

EP 0 298 405 A2

Xerox Copy Centre

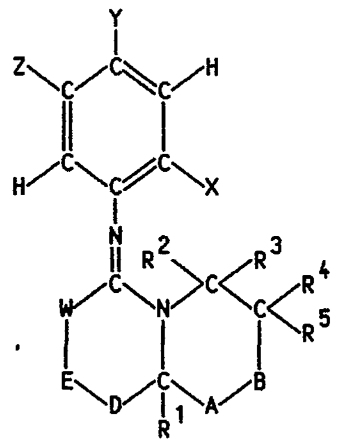

(I) ,

in der A, B, D, E, W, X, Y, Z, $R^1$, $R^2$, $R^3$, $R^4$ und $R^5$ die in der Beschreibung genannten Bedeutungen haben, Verfahren zu ihrer Herstellung und ihre Verwendung als Mittel mit herbizider Wirkung.

# ANELLIERTE IMINO-AZOLE UND IMINO-AZINE, VERFAHREN ZU IHRER HERSTELLUNG UND IHRE VERWENDUNG ALS MITTEL MIT HERBIZIDER WIRKUNG

Die Erfindung betrifft neue anellierte Imino-azole und Imino-azine, Verfahren zu ihrer Herstellung und ihre Verwendung als Mittel mit herbizider Wirkung.

Es ist bereits bekannt, daß Phenyliminodiazolverbindungen herbizide Eigenschaften besitzen (DE-OS 36 28 583). Häufig ist jedoch die Herbizidwirkung der bekannten Verbindungen nicht ausreichend, oder es treten Selektivitätsprobleme in wichtigen landwirtschaftlichen Kulturen auf.

Aufgabe der vorliegenden Erfindung ist daher die Bereitstellung von neuen Verbindungen, die diese Nachteile nicht aufweisen und in ihren biologischen Eigenschaften den bisher bekannten Verbindungen überlegen sind.

Es wurde gefunden, daß Imino-azole und Imino-azine der allgemeinen Formel I

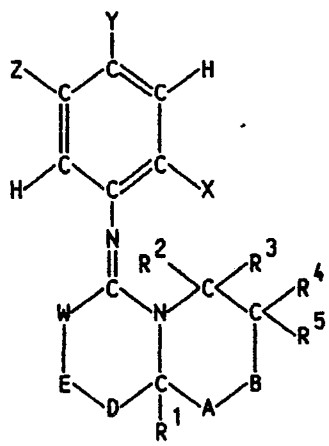

(I) ,

in der

A die Gruppe $(CR^6R^7)_n$,

B ein Sauerstoffatom oder die Gruppe $CR^8R^9$,

D die Gruppe $(CR^{10}R^{11})_n$,

E die Gruppe $CR^{12}R^{13}$,

W ein Sauerstoffatom oder die Gruppe $S(O)_m$,

X und Y unabhängig voneinander ein Wasserstoff- oder Halogenatom,

Z ein Wasserstoff- oder Halogenatom, einen Trihalogenmethylrest oder eine der Gruppen $-OR^{14}$, $-SR^{14}$, $-NR^{14}_2$ oder $-CO_2R^{15}$,

$R^1$ ein Wasserstoffatom, einen $C_1$-$C_2$-Alkyl- oder Halogen-$C_1$-$C_2$-alkylrest,

$R^2$, $R^3$, $R^4$, $R^5$, $R^6$, $R^7$, $R^8$, $R^9$, $R^{10}$, $R^{11}$, $R^{12}$ und $R^{13}$ unabhängig voneinander ein Wasserstoffatom, einen $C_1$-$C_4$-Alkylrest, einen Halogen-$C_1$-$C_4$-alkylrest, einen $C_1$-$C_4$-Alkoxyrest, einen $C_1$-$C_4$-Alkylthiorest, einen Halogen-$C_1$-$C_4$-alkoxyrest oder einen Halogen-$C_1$-$C_4$-alkylthiorest oder

$R^2$, $R^3$, $R^4$, $R^5$, $R^6$, $R^7$, $R^8$, $R^9$, $R^{10}$, $R^{11}$, $R^{12}$ und $R^{13}$ gegebenenfalls zusammen ein oder mehrere Ringe oder Alkylidengruppen,

$R^{14}$ ein Wasserstoffatom, einen gegebenenfalls durch ein oder mehrere Halogenatome substituierten $C_1$-$C_{12}$-Alkyl-, $C_3$-$C_8$-Cycloalkyl-, $C_2$-$C_6$-Alkenyl-oder $C_3$-$C_8$-Alkinylrest, einen Hydroxy-carbonyl-$C_1$-$C_8$-alkylrest, einen $C_1$-$C_4$-Alkoxy-, $C_2$-$C_4$-Alkenyloxy- oder $C_3$-$C_4$-Alkinyloxycarbonyl-$C_1$-$C_8$-alkylrest, einen $C_1$-$C_8$-Alkoxy-$C_1$-$C_8$-alkylrest, einen gegebenenfalls durch ein oder mehrere Halogenatome substituierten $C_1$-$C_4$-Alkylsulfonylrest, einen gegebenenfalls ein oder mehrfach, gleich oder verschieden durch Halogen, $C_1$-$C_4$-Alkyl, Halogen-$C_1$-$C_4$-alkyl, Nitro, $C_1$-$C_4$-Alkoxy oder Halogen-$C_1$-$C_4$-Alkoxy substituierten Phenylsulfonylrest, einen durch einen gesättigten oder ungesättigten Heterocyclus substituierten $C_1$-$C_4$-Alkylrest oder einen gegebenenfalls ein-oder mehrfach, gleich oder verschieden durch Halogen, $C_1$-$C_4$-Alkyl, Halogen-$C_1$-$C_4$-Alkyl, Nitro, $C_1$-$C_4$-Alkoxy oder Halogen-$C_1$-$C_4$-Alkoxy substituierten Phenyl- oder Phenyl-$C_1$-$C_4$-alkylrest,

$R^{15}$ ein Wasserstoffatom, einen gegebenenfalls durch ein oder mehrere Halogenatome substituierten $C_1$-$C_6$-Alkyl-, $C_2$-$C_6$-Alkenyl- oder $C_3$-$C_6$-Alkinylrest,

n 0 oder 1 und
m 0, 1 oder 2
bedeuten, und deren Salze mit anorganischen und organischen Säuren, eine interessante herbizide Wirkung zeigen.

Die Verbindungen der allgemeinen Formel I können gegebenenfalls in verschiedenen enantiomeren, diastereomeren oder geometrischen Formen anfallen und sind ebenfalls Gegenstand dieser Erfindung.

Die Bezeichnung "Halogen" umfaßt Fluor, Chlor, Brom und Jod.

Die Bezeichnung "Halogenalkyl" besagt, daß ein oder mehrere Wasserstoffatome des Alkylrestes durch Halogen ersetzt sind.

Unter einem gesättigten oder ungesättigten Heterocyclus sind zum Beispiel zu verstehen Tetrahydrofuran, Tetrahydrothiophen, Pyrrolidin, Piperidin, Morpholin, Pyridin, Pyrrol.

Die erfindungsgemäßen Verbindungen der allgemeinen Formel I lassen sich herstellen, indem man

A) eine Verbindung der allgemeinen Formel II

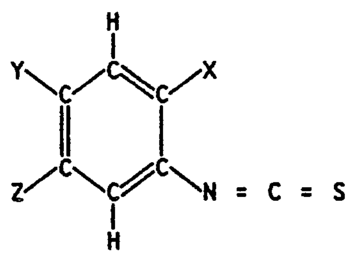

(II) ,

in der X, Y und Z die unter der allgemeinen Formel I angegebenen Bedeutungen haben, in einem Eintopfverfahren mit einer Verbindung der allgemeinen Formel III

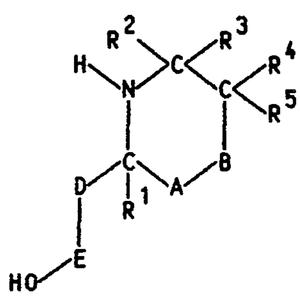

(III) ,

in der A, B, D, E, $R^1$, $R^2$, $R^3$, $R^4$ und $R^5$ die unter der allgemeinen Formel I angegebenen Bedeutungen haben, unter sauren Bedingungen cyclisiert.

B) falls in der allgemeinen Formel I W für ein Sauerstoffatom steht, eine Verbindung der allgemeinen Formel II

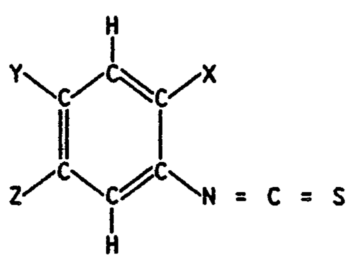

(II) ,

in der X, Y und Z die unter der allgemeinen Formel I angegebenen Bedeutungen haben, in einem Eintopfverfahren mit einer Verbindung der allgemeinen Formel III

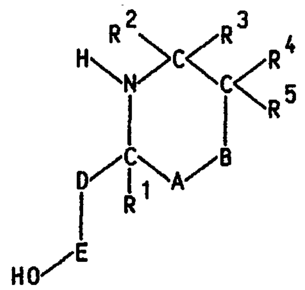

(III) ,

in der A, B, D, E, $R^1$, $R^2$, $R^3$, $R^4$ und $R^5$ die unter der allgemeinen Formel I angegebenen Bedeutungen haben, in einem geeigneten Lösungsmittel umsetzt,

C) einen Thioharnstoff der allgemeinem Formel IV

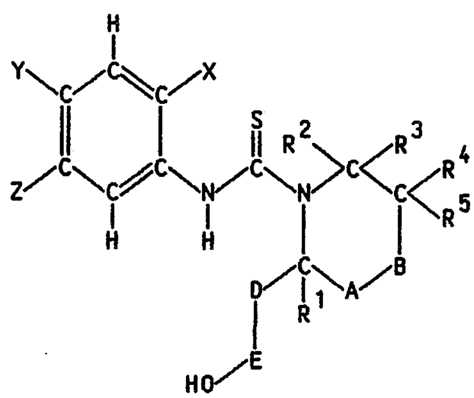

(IV) ,

in der A, B, D, E, X, Y, Z, $R^1$, $R^2$, $R^3$, $R^4$ und $R^5$ die unter der allgemeinen Formel I angegebenen Bedeutungen haben, unter sauren Bedingungen oder gegebenenfalls in einem geeigneten Lösungsmittel unter neutralen Bedingungen cyclisiert.

D) eine Verbindung der allgemeinen Formel V

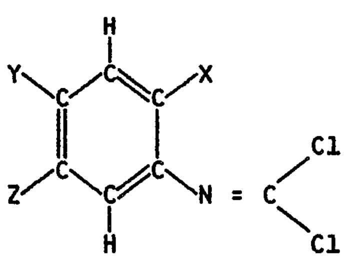

(V) ,

in der X, Y und Z die unter der allgemeine Formel I angegebenen Bedeutungen haben, mit einer Verbindung der allgemeinen Formel VI

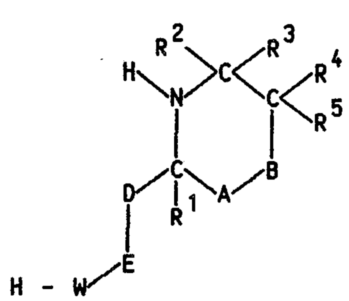

(VI) ,

in der A, B, D, E, W, $R^1$, $R^2$, $R^3$, $R^4$ und $R^5$ die unter der allgemeinen Formel I angegebenen Bedeutungen

haben, in einem geeigneten Lösungsmittel unter basischen Bedingungen umsetzt,

E) falls in der allgemeinen Formel I Z für eine der Gruppen $-OR^{14}$, $-SR^{14}$ oder $-NR_2^{14}$ steht, $R^{14}$ aber nicht ein Wasserstoffatom, einen Phenylrest oder einen substituierten Phenylrest darstellt, eine Verbindung der allgemeinen Formel VII

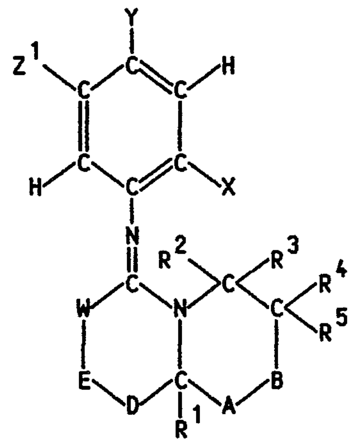

(VII) ,

in der A, B, D, E, W, X, Y, $R^1$, $R^2$, $R^3$, $R^4$ und $R^5$ die unter der allgemeinen Formel I angegebenen Bedeutungen haben und $Z^1$ für die Gruppen -OH, -SH oder $-NH_2$ steht, mit Verbindungen der allgemeinen Formel VIII

$R^{14}$ - G (VIII) ,

in der G für ein Halogenatom, einen p-Toluolsulfonyloxy- oder Methansulfonyloxyrest oder die Gruppe $-O-SO_2-OR^{14}$ steht und $R^{14}$ die unter der allgemeinen Formel I angegebene Bedeutung hat, aber nicht ein Wasserstoffatom, einen Phenylrest oder einen substituierten Phenylrest darstellt, gegebenenfalls unter Zusatz einer Base umsetzt,

F) falls in der allgemeinen Formel I Z für die Gruppe -OH steht, eine Verbindung der allgemeinen Formel IX

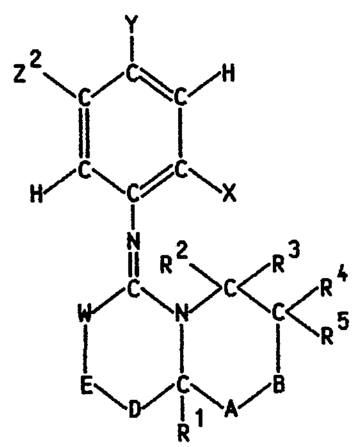

(IX) ,

in der A, B, D, E, W, X, Y, $R^1$, $R^2$, $R^3$, $R^4$ und $R^5$ die unter der allgemeinen Formel I angegebenen Bedeutungen haben und $Z^2$ für einen $C_1$-$C_{14}$-Alkyloxyrest oder einen $C_3$-$C_8$-Cycloalkyloxyrest steht, mit einer starken Mineralsäure einer Etherspaltung unterzieht oder

G) falls in der allgemeinen Formel I Z für eine der Gruppen -OH, -SH oder $-NH_2$ steht, eine Verbindung der allgemeinen Formel X

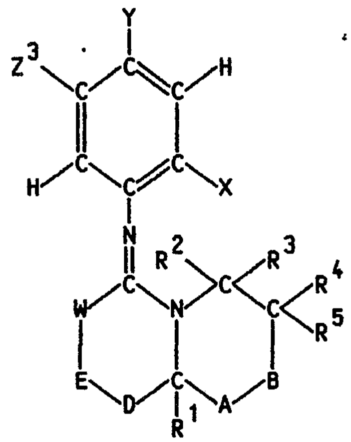

(X) ,

in der A, B, D, E, W, X, Y, $R^1$, $R^2$, $R^3$, $R^4$ und $R^5$ die unter der allgemeinen Formel I angegebenen Bedeutungen hat und $Z^3$ für eine der Gruppen -OR, -SR oder $NR_2$ mit R in der Bedeutung eines gegebenenfalls durch ein oder mehrere Halogenatome substituierten $C_1$-$C_4$-Alkylsulfonylrestes oder eines gegebenenfalls durch $C_1$-$C_3$-Alkyl oder Halogen substituierten Phenylsulfonylrestes steht, basisch hydrolysiert.

Die hier genannten Ausgangsmaterialien sind, sofern die Herstellung nicht beschrieben ist, bekannt oder lassen sich analog zu bekannten Verfahren herstelllen.

Die Verfahrensvariante A) wird zweckmäßigerweise so durchgeführt, daß man die Ausgangsverbindungen der allgemeinen Formeln II und III in einem organischen Lösungsmittel, wie zum Beispiel Diethylether, Tetrahydrofuran, Dioxan, Methanol oder Ethanol, gegebenenfalls unter Zusatz katalytischer Mengen eines Amins, wie zum Beispiel Triethylamin, über längere Zeit, wie zum Beispiel 0,5 bis 10 Stunden, bei einer Temperatur von 20° C bis zur Siedetemperatur des jeweiligen Lösungsmittels umsetzt. Danach wird das Lösungsmittel entfernt und der Rückstand mit einer starken Mineralsäure, wie zum Beispiel Salzsäure, Bromwasserstoffsäure oder auch Schwefelsäure, 0,5 bis 10 Stunden zum Sieden erhitzt. Nach dem Neutralisieren mit zum Beispiel Natronlauge wird wie üblich aufgearbeitet. Die Salze der Verbindungen werden erhalten, wenn man vor dem Neutralisieren nach üblichen Verfahren aufarbeitet.

Die Verfahrensvariante B) wird zweckmäßigerweise so durchgeführt, daß man die Ausgangsverbindungen der allgemeinen Formeln II und III in einem wie unter Verfahren A angegebenen organischen Lösungsmittel gegebenenfalls unter Zusatz katalytischer Mengen eines Amins, wie zum Beispiel Triethylamin, über längere Zeit, wie zum Beispiel 0,5 bis 15 Stunden, bei einer Temperatur von 20° C bis zur Siedetemperatur, gewöhnlich aber bei der Siedetemperatur des jeweiligen Lösungsmittels, umsetzt.

Die Verfahrensvariante C) wird zweckmäßigerweise so durchgeführt, daß man Thioharnstoffe der allgemeinen Formel IV mit einer starken Mineralsäure, wie zum Beispiel Salzsäure, Bromwasserstoffsäure oder auch Schwefelsäure, 0,5 bis 10 Stunden zum Sieden erhitzt. Die Cyclisierung kann gegebenenfalls auch unter neutralen Bedingungen erfolgen, indem man die Thioharnstoffe der allgemeinen Formel IV in einem wie unter Verfahren A angegebenen organischen Lösungsmittel längere Zeit, wie zum Beispiel 0,5 bis 15 Stunden, erhitzt. Die Temperatur kann zwischen 50° C und der Siedetemperatur des entsprechenden Lösungsmittels variieren.

Die Verfahrensvariante D) wird zweckmäßigerweise so durchgeführt, daß man Verbindungen der allgemeinen Formel V mit Verbindungen der allgemeinen Formel VI in einem geeigneten Lösungsmittel in Gegenwart von Basen bei Temperaturen zwischen 0° C und 100° C, vorzugsweise zwischen 10° C und 50° C umsetzt. Als Lösungsmittel kommen alle inerten organischen Lösungsmittel in Frage. Hierzu gehören vorzugsweise Kohlenwaserstoffe, wie zum Beispiel Toluol, chlorierte Kohlenwasserstoffe, wie zum Beispiel Methylenchlorid, Chloroform oder Tetrachlorkohlenstoff, und Ether, wie Diethylether, Dioxan oder Tetrahydrofuran. Als Basen können alle anorganischen und organischen Basen, vorzugsweise aber organische Basen, wie zum Beispiel Triethylamin oder Pyridin, verwendet werden.

Die Verfahrensvariante E) wird zweckmäßigerweise so durchgeführt, daß man die Ausgangsmaterialien in einem geeigneten Lösungsmittel unter Zugabe einer anorganischen oder organischen Base bei Temperaturen zwischen 0° C und 150° C, bevorzugt abver bei Rückflußtemperatur des Lösungsmittels, zur Reaktion bringt. Die Reaktion kann auch gegebenenfalls in einem Zweiphasensystem unter Zugabe eines Phasentransferkatalysators durchgeführt werden.

Als Basen können Alkali- und Erdalkalihydroxide, Alkoholate, Alkalihydride, Alkali- und Erdalkalicarbonate und -hydrogencarbonate, tertiäre aliphatische und aromatische Amine sowie heterocyclische Basen

eingesetzt werden. Beispielhaft seien Natrium- und Kaliumhydroxid, Natriummethanolat, Natriumhydrid, Natrium- und Kaliumcarbonat, Natrium- und Kaliumhydrogencarbonat, Triethylamin und Pyridin genannt.

Als Lösungsmittel kommen Kohlenwasserstoffe, wie zum Beispiel Toluol, chlorierte Kohlenwasserstoffe, wie zum Beispiel Methylenchlorid oder Chloroform, Ether, wie zum Beispiel Diethylether oder Tetrahydrofuran, Alkohole, wie zum Beispiel Methanol oder Ethanol, Ketone, wie zum Beispiel Aceton oder Butanon, Amide, wie zum Beispiel Dimethylformamid, oder auch Sulfoxide, wie zum Beispiel Dimethylsulfoxid, in Frage.

Die Verfahrensvariante F) wird zweckmäßigerweise so durchgeführt, daß man den Phenolether mit einer starken Mineralsäure erhitzt. Als Säuren kommen zum Beispiel Bromwasserstoffsäure oder Jodwasserstoffsäure in Frage. Gewöhnlich dient die Säure gleichzeitig als Lösungsmittel, es können aber auch organische Säuren, wie zum Beispiel Eisessig, zugesetzt werden. Die Umsetzung erfolgt bei einer Temperatur von 50 °C bis zur Siedetemperatur des jeweiligen Lösungsmittels, gewöhnlich aber bei der Siedetemperatur des Lösungsmittels.

Die Verfahrensvariante G) wird zweckmäßigerweise so durchgeführt, daß man das Ausgangsmaterial mit einer wäßrigen oder wäßrig-alkoholischen Alkali-oder Erdalkalihydroxid-, Alkali- oder Erdalkalicarbonat- oder -hydrogencarbonat-Lösung bei Temperaturen von 20 °C bis 150 °C zu den entsprechenden Derivaten hydrolisiert.

Die Aufarbeitung der nach den Verfahrensvarianten A) bis G) hergestellten erfindungsgemäßen Verbindungen erfolgt in der üblichen Art und Weise. Eine Aufreinigung oder gegebenenfalls auch Trennung eventuell anfallender isomerer Verbindungen erfolgt durch Kristallisation oder Säulenchromatographie.

Die erfindungsgemäßen Wirkstoffe zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden. Die erfindungsgemäßen Wirkstoffe können auch als Defoliants, als Desiccants und als Krautabtötungsmittel verwendet werden.

Die erfindungsgemäßen Wirkstoffe können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monocharia, Fimbristylis, Eleocharis, Ischaemum und Apera.

Die Aufwandmengen schwanken je nach Anwendungsart im Vor- und Nachauflauf in den Grenzen zwischen 0,05 und 5 kg/ha.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 35, No.5, 1986, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie

Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Bensolzulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 25 Gewichtsprozent Wirkstoff
60 Gewichtsprozent Kaolin
10 Gewichtsprozent Kieselsäure
5 Gewichtsprozent einer Mischung aus dem Calciumsalz der Ligninsulfonsäure und dem Natriumsalz des N-Methyl-N-oleyl-taurins
2.) 40 Gewichtsprozent Wirkstoff
25 Gewichtsprozent Tonmineralien
25 Gewichtsprozent Kieselsäure
10 Gewichtsprozent einer Mischung aus dem Calciumsalz der Ligninsulfonsäure und Alkylphenylpolyglycolethern

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

25 Gewichtsprozent Wirkstoff
15 Gewichtsprozent Cyclohexanon
55 Gewichtsprozent Xylol
5 Gewichtsprozent einer Mischung aus dem Calciumsalz der Dodecylbenzolsulfonsäure und Nonylphenylpolyoxyethylen

Die nachfolgenden Beispiele erläutern die Herstellung der erfindungsgemäßen Verbindungen:

**Beispiel 1**

3-(4-Chlor-2-fluor-5-methylsulfonyloxyphenylimino)-tetrahydro-1H,3H-pyrrolo[1,2-c]thiazol (Verfahren A)

10 g 2-(Hydroxymethyl)-pyrrolidin und 27,6 g 4-Chlor-2-fluor-5-methylsulfonyloxyphenylisothiocyanat werden 4 Stunden in 200 ml Dioxan unter Rückfluß erhitzt. Das Lösungsmittel wird abgezogen und der Rückstand mit 50 ml 48%iger Bromwasserstoffsäure 2 Stunden zum Sieden erhitzt. Nach dem Abkühlen gibt man die Reaktionslösung auf eine Eis-Wasser-Mischung, neutralisiert mit 40%iger Natronlauge und extrahiert mit Methylenchlorid. Nach dem Trocknen und Einengen wird das Rohprodukt durch Säulenchromatographie gereinigt (Kieselgel, Elutionsmittel: Hexan/Essigester).
Ausbeute: 31 g = 87 % der Theorie
Fp.: 68-70 °C

**Beispiel 2**

3-(4-Chlor-2-fluor-5-methoxyphenylimino)-1-methyl-hexahydro-3H-oxazolo[3,4-a]pyridin (Verfahren B)

14,3 g 2-(1-Hydroxyethyl)-piperidin in 25 ml Dioxan werden bei Raumtemperatur mit 23,9 g 4-Chlor-2-fluor-5-methoxyphenylisothiocyanat versetzt und 4 Stunden unter Rückfluß erhitzt. Das Lösungsmittel wird entfernt und das Rohprodukt durch Säulenchromatographie gereinigt (Kieselgel, Elutionsmittel: Hexan/Essigester).
Ausbeute: 34,9 g = 41 % der Theorie
Fp.: zähes Öl

**Beispiel 3**

3-(2-Chlorphenylimino)-hexahydro-3H-thiazolo[3,4-a]pyridin (Verfahren C)

1 g 1-(2-Chlorphenyl-thiocarbamoyl)-2-hydroxymethylpiperidin in 20 ml 48%iger Bromwasserstoffsäure werden 2 Stunden unter Rückfluß erhitzt. Danach wird unter Kühlung mit 2 N Natronlauge neutralisiert, mit Methylenchlorid extrahiert, über Magnesiumsulfat getrocknet und das Lösungsmittel abdestilliert. Der Rückstand wird aus Isopropylether umkristallisiert.
Ausbeute: 0,64 g = 68 % der Theorie
Fp.: 110 °C

Das Ausgangsmaterial wird wie folgt hergestellt:

1-(2-Chlorphenyl-thiocarbamoyl)-2-hydroxymethyl-piperidin

3,4 g 2-Hydroxymethylpiperidin in 50 ml absolutem Tetrahydrofuran werden mit einem Tropfen Triethylamin und 5 g 2-Chlor-phenylisothiocyanat versetzt. Nach 12 Stunden Stehen bei Raumtemperatur wird die Reaktionslösung im Eisbad abgekühlt. Die Kristalle werden abgesaugt und aus Essigester umkristallisiert.
Ausbeute: 3,5 g = 45 % der Theorie
Fp.: 148 °C

**Beispiel4**

3-Phenylimino-hexahydro-3H-oxazolo[3,4-a]pyridin (Verfahren D)

3 g Phenylisocyaniddichlorid in 10 ml Methylenchlorid werden bei Raumtemperatur mit einer Lösung von 5,4 ml Triethylamin und 1,7 g 2-Hydroxymethylpiperidin in 20 ml Methylenchlorid versetzt. Nach 12 Stunden Stehen bei Raumtemperatur wird mit Wasser gewaschen, die organische Phase über Magnesiumsulfat getrocknet, das Lösungsmittel abdestilliert und der Rückstand aus Isopropylether umkristallisiert.
Ausbeute: 1,7 g = 52 % der Theorie
Fp.: 58 °C

**Beispiel 5**

3-(4-Chlor-2-fluor-5-isopropoxyphenylimino)-1-methyl-hexahydro-3H-oxazolo[3,4-a]pyridin (Verfahren E)

3,5 g 3-(4-Chlor-2-fluor-5-hydroxyphenylimino)-1-methyl-hexahydro-3H-oxazolo[3,4-a]pyridin und 6,6 g Kaliumcarbonat in 40 ml Aceton werden bei Raumtemperatur mit 3,1 g 2-Brompropan versetzt und 6 Stunden unter Rückfluß erhitzt. Danach werden erneut 3,1 g 2-Brompropan zugesetzt und 6 Stunden unter Rückfluß erhitzt. Nach dem Abkühlen wird filtriert, das Filtrat eingeengt und der Rückstand durch Säulenchromatographie gereinigt (Kieselgel, Elutionsmittel: Hexan/Essigester).
Ausbeute: 3,7 g = 93 % der Theorie
Fp.: Öl ($n_D^{40}$ 1,5502)

**Beispiel 6**

3-(4-Chlor-2-fluor-5-hydroxyphenylimino)-1-methyl-hexahydro-3H-oxazolo[3,4-a]pyridin (Verfahren F)

14,4 g 3-(4-Chlor-2-fluor-5-methoxyphenylimino)-1-methyl-hexahydro-3H-oxazolo[3,4-a]pyridin werden mit 25 ml 48%iger Bromwasserstoffsäure 5 Stunden unter Rückfluß erhitzt. Nach dem Abkühlen wird mit 40%iger Natronlauge neutralisiert und mit Essigester extrahiert. Die organische Phase wird über Magnesiumsulfat getrocknet, eingeengt und der Rückstand aus Petrolether umkristallisiert.
Ausbeute: 8 g = 58 % der Theorie
Fp.: 144 °C

**Beispiel 7**

3-(4-Chlor-2-fluor-5-hydroxyphenylimino)-tetrahydro-1H,3H-pyrrolo[1,2-c]thiazol (Verfahren G)

22 g 3-(4-Chlor-2-fluor-5-methylsulfonyloxyphenylimino)-tetrahydro-1H,3H-pyrrolo[1,2-c]thiazol in 400 ml Ethanol werden mit 400 ml 2 N Natronlauge versetzt und 4 Stunden bei 40 °C gerührt. Unter Eiskühlung wird mit 10%iger Salzsäure neutralisiert und mit Methylenchlorid extrahiert. Nach dem Trocknen über Magnesiumsulfat und Einengen wird der Rückstand aus Isopropanol umkristallisiert.
Ausbeute: 1,58 g = 91 % der Theorie
Fp.: 158-161 °C

Analog zu den Beispielen 1 bis 7 wurden auch die folgenden Beispiele hergestellt:

| Beispiel Nr. | $R^1$ | $R^2$ | $R^3$ | $R^4$ | $R^5$ | A | B | D | E | W | X | Y | Z | Fp. (°C) bzw. $n_D$ |
|---|---|---|---|---|---|---|---|---|---|---|---|---|---|---|
| 8 | H | H | H | H | H | $CH_2$ | $CH_2$ | $CH_2$ | $CH_2$ | S | F | Cl | $OCH_3$ | 79-81 |
| 9 | H | H | H | H | H | $CH_2$ | $CH_2$ | - | $CH_2$ | S | F | Cl | $OCH_3$ | Öl |
| 10 | H | H | H | H | H | - | $CH_2$ | - | $CH_2$ | S | F | Cl | $OCH_3$ | 118 |
| 11 | H | H | H | H | H | - | $CH_2$ | - | $CH_2$ | S | F | Cl | OH | 58-161 |
| 12 | H | H | H | H | H | - | $CH_2$ | - | $CH_2$ | S | F | Cl | $OCH(CH_3)_2$ | 82-84 |
| 13 | H | H | H | H | H | $CH_2$ | $CH_2$ | - | $CH_2$ | O | F | Cl | OH | 160 |
| 14 | H | H | H | H | H | $CH_2$ | $CH_2$ | - | $CH_2$ | O | F | Cl | $OSO_2CH_3$ | 96-98 |
| 15 | H | H | H | H | H | $CH_2$ | $CH_2$ | - | $CH_2$ | O | F | Cl | $OCH(CH_3)_2$ | $n_D^{22}$ = 1,5618 |
| 16 | H | H | H | H | H | $CH_2$ | $CH_2$ | - | $CHCH_3$ | O | F | Cl | $OCH_2CO_2C_2H_5$ | 95 |
| 17 | H | H | H | H | H | $CH_2$ | $CH_2$ | - | $CHCH_2CH_3$ | O | F | Cl | OH | 162 |
| 18 | H | H | H | H | H | $CH_2$ | $CH_2$ | - | $CHCH_2CH_3$ | O | F | Cl | $OCH_3$ | Öl |
| 19 | H | $CH_3$ | H | H | H | $CH_2$ | $CH_2$ | - | $CHCH_3$ | O | F | Cl | $OCH_3$ | 78 |
| 20 | H | H | H | H | H | $CH_2$ | $CH_2$ | - | $CH_2$ | S | F | Cl | OH | 138-140 |
| 21 | H | H | H | H | H | - | $CH_2$ | - | $CH_2$ | S | F | Cl | $OCH(CH_3)C_2H_5$ | 72-74 |
| 22 | H | H | H | H | H | $CH_2$ | $CH_2$ | - | $CH_2$ | S | F | Cl | $OSO_2CH_3$ | 96-98 |
| 23 | H | H | H | H | H | $CH_2$ | $CH_2$ | - | $CH_2$ | S | F | Cl | $OCH(CH_3)C_2H_5$ | 55-58 |
| 24 | H | H | H | H | H | $CH_2$ | $CH_2$ | $CH_2$ | $CH_2$ | S | F | Cl | $OSO_2CH_3$ | 100 |
| 25 | H | H | H | H | H | $CH_2$ | $CH_2$ | $CH_2$ | $CH_2$ | S | F | Cl | OH | 138 |
| 26 | H | H | H | H | H | $CH_2$ | $CH_2$ | $CH_2$ | $CH_2$ | S | F | Cl | OH | >220 (xHCl) |
| 27 | H | H | H | H | H | $CH_2$ | $CH_2$ | - | $CH_2$ | S | F | Cl | $OCH_2CO_2C_2H_5$ | 93 |
| 28 | H | H | H | H | H | $CH_2$ | $CH_2$ | - | $CH_2$ | S | F | Cl | $OCH_2CH=CH_2$ | $n_D^{42}$ = 1,596 |
| 29 | H | H | H | H | H | $CH_2$ | $CH_2$ | - | $CH_2$ | S | F | Cl | $OCH_2C\equiv CH$ | 77-79 |
| 30 | H | H | H | H | H | $CH_2$ | $CH_2$ | - | $C(CH_3)_2$ | O | F | Cl | $OCH_3$ | Öl |
| 31 | H | H | H | H | H | $CH_2$ | $CH_2$ | - | $CH_2$ | O | Cl | H | H | 45-48 |
| 32 | H | H | H | H | H | - | $CH_2$ | - | $CH_2$ | S | F | Cl | $OCH_2CH=CH_2$ | Öl |
| 33 | H | H | H | H | H | $CH_2$ | $CH_2$ | - | $CH_2$ | S | F | Cl | $OCH(CH_3)_2$ | $n_D^{42}$ = 1,580 |

| Beispiel Nr. | $R^1$ | $R^2$ | $R^3$ | $R^4$ | $R^5$ | A | B | D | E | W | X | Y | Z | Fp. (°C) bzw. $n_D$ |
|---|---|---|---|---|---|---|---|---|---|---|---|---|---|---|
| 34 | H | H | H | $CH_3$ | $CH_3$ | - | $CH_2$ | - | $CH_2$ | S | F | Cl | $OCH_3$ | Öl |
| 35 | H | H | H | H | H | - | $CH_2$ | - | $CH_2$ | S | F | Cl | $OCH_2C{\equiv}CH$ | 105 |
| 36 | H | H | H | H | H | - | $CH_2$ | - | $CH_2$ | S | F | Cl | $OCH_2CO_2C_2H_5$ | Öl |
| 37 | H | H | H | H | H | - | $CH_2$ | - | $CH_2$ | O | F | Cl | $OCH(CH_3)_2$ | Öl |
| 38 | H | H | H | H | H | - | $CH_2$ | - | $CH_2$ | O | F | Cl | $O(CH_2)_3CH_3$ | Öl |
| 39 | H | H | H | H | H | - | $CH_2$ | - | $CH_2$ | O | F | Cl | $OCH_2CO_2C_2H_5$ | Öl |
| 40 | H | H | H | H | H | - | $CH_2$ | - | $CH_2$ | S | F | Cl | $CO_2C_2H_5$ | $n_D^{20}$ = 1,5978 |
| 41 | H | H | H | H | H | $CH_2$ | $CH_2$ | - | $CH_2$ | S | F | Cl | $CO_2C_2H_5$ | 51 |
| 42 | H | H | H | H | H | $CH_2$ | $CH_2$ | - | $CH_2$ | S | Cl | Cl | H | 61 |
| 43 | H | H | H | H | H | $CH_2$ | $CH_2$ | - | $CH_2$ | S | H | H | $CF_3$ | 83 |
| 44 | H | H | H | H | H | $CH_2$ | $CH_2$ | - | $CH_2$ | S | F | Cl | $CO_2H$ | 96 (Zers.) |
| 45 | H | H | H | H | H | - | $CH_2$ | - | $CH_2$ | S | F | Cl | $CO_2H$ | 97 (Zers.) |
| 46 | H | H | H | H | H | $CH_2$ | $CH_2$ | - | $CH_2$ | S | F | Cl | $CO_2CH_3$ | $n_D^{20}$ = 1,6076 |
| 47 | H | H | H | H | H | - | $CH_2$ | - | $CH_2$ | S | F | Cl | $CO_2CH_3$ | $n_D^{20}$ = 1,6171 |
| 48 | H | H | H | H | H | $CH_2$ | $CH_2$ | - | $CH_2$ | S | F | Cl | $CO_2CH_2C{\equiv}CH$ | $n_D^{20}$ = 1,6081 |
| 49 | H | H | H | H | H | - | $CH_2$ | - | $CH_2$ | S | F | Cl | $CO_2CH_2C{\equiv}CH$ | 86 |
| 50 | H | H | H | H | H | $CH_2$ | $CH_2$ | - | $CH_2$ | S | F | Cl | $CO_2(CH_2)_4CH_3$ | $n_D^{20}$ = 1,5753 |
| 51 | H | H | H | H | H | - | $CH_2$ | - | $CH_2$ | S | F | Cl | $CO_2(CH_2)_4CH_3$ | $n_D^{20}$ = 1,5826 |
| 52 | H | H | H | H | H | $CH_2$ | $CH_2$ | - | $CH_2$ | S | F | Cl | $SCH_2CO_2$- 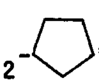 | Harz |
| 53 | H | H | H | H | H | $CH_2$ | $CH_2$ | - | $CH_2$ | S | Cl | F | $CO_2CH_3$ | $n_D^{20}$ = 1,5912 |

Die folgenden Beispiele erläutern die Anwendungsmöglichkeiten der erfindungsgemäßen Verbindungen:

Beispiel A

Im Gewächshaus wurden die Aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsion mit 500 Litern Wasser/ha gleichmäßig über die Pflanzen versprüht. Hier zeigten drei Wochen nach der Behandlung die erfindungsgemäßen Verbindungen eine hohe Kulturpflanzenselektivität bei ausgezeichneter Wirkung gegen das Unkraut.

In der folgenden Tabelle bedeuten:

0 = nicht geschädigt
4 = total vernichtet
- = nicht geprüft

Br = Brassica napus napus
Ho = Hordeum vulgare
Or = Oryza sativa
So = Solanum ssp.
Ph = Phaseolus vulgaris
He = Helianthus annuus
Ab = Abutilon hybridum
Ma = Matricaria chamomilla
Vi = Viola tricolor
Ch = Chrysanthemum segetum
Ip = Ipomoea purpurea
Se = Setaria italica
Ga = Galium aparine

| Erfindungsgemäße Verbindung | Br | Ho | Or | So | Ph | He | Ab | Ma | Vi | Ch | Ip | Se | Ga |
|---|---|---|---|---|---|---|---|---|---|---|---|---|---|
| Beispiel 10 | 0 | 0 | 0 | 4 | 4 | 3 | 4 | 3 | 4 | 3 | 3 | 3 | 3 |
| Beispiel 34 | - | 0 | - | - | - | - | 4 | 2 | - | - | 4 | 2 | 3 |
| Beispiel 37 | 0 | 0 | - | - | - | - | 4 | 0 | 3 | - | 3 | - | 2 |
| Beispiel 40 | - | 0 | - | - | - | - | 4 | 3 | - | - | 4 | 3 | 4 |
| Beispiel 41 | - | 1 | - | - | - | - | 4 | 4 | - | - | 4 | 3 | 4 |
| Unbehandelt | 0 | 0 | 0 | 0 | 0 | 0 | 0 | 0 | 0 | 0 | 0 | 0 | 0 |

Die gleiche herbizide Wirkung zeigten auch die erfindungsgemäßen Verbindungen der Beispiele 1 bis 9, 11 bis 33, 35, 36, 38, 39 und 42 bis 53.

Beispiel

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsion mit 500 Litern Wasser/ha gleichmäßig über die Pflanzen versprüht. Hier zeigten zwei Wochen nach der Behandlung die erfindungsgemäßen Verbindungen eine hohe Kulturpflanzenselektivität in Weizen bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung

Be = Beta vulgaris altissima
Br = Brassica sp.

Tr = Triticum aestivum
Se = Setaria viridis
Ga = Galium aparine
Po = Polygonum sp.
Ve = Veronica persica
Vi = Viola sp.

| Erfindungsgemäße Verbindungen | Tr | Be | Br | Se | Ga | Po | Ve | Vi |
|---|---|---|---|---|---|---|---|---|
| Beispiel 35 | 0 | 4 | 4 | 3 | 4 | 4 | 4 | 4 |
| Beispiel 47 | 0 | 4 | 4 | 4 | 4 | 4 | 4 | 4 |
| Beispiel 51 | 0 | 4 | 4 | 3 | 4 | 4 | 4 | 4 |
| Unbehandelt | 0 | 0 | 0 | 0 | 0 | 0 | 0 | 0 |
| Vergleichsmittel | | | | | | | | |
| Ioxynil | 0 | 4 | 3 | 0 | 0 | 2 | 3 | 2 |

Beispiel C

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsion mit 500 Litern Wasser/ha gleichmäßig über die Pflanzen versprüht. Hier zeigten zwei Wochen nach der Behandlung die erfindungsgemäßen Verbindungen eine hohe Kulturpflanzenselektivität in Mais bei ausgezeichneter Wirkung gegen das Unkraut. Das Vergleichsmittel zeigte nicht die gleichhohe Selektivität.

In der folgenden Tabelle bedeuten:
0 = keine Schädigung
1 = 1 - 24 % Schädigung
2 = 25 - 74 % Schädigung
3 = 75 - 89 % Schädigung
4 = 90 - 100 % Schädigung
\- = nicht geprüft

Ze = Zea mays
Be = Beta vulgaris altissima
Br = Brassica sp.
Gl = Glycine maxima
Go = Gossypium hirsutum
He = Helianthus annuus
Se = Setaria viridis
Ab = Abutilon theophrasti
Ga = Galium aparine
Ip = Ipomoea purpurea
Ma = Matricaria chamomilla
Po = Polygonum sp.
Sb = Sesbania exaltata
So = Solanum sp.
Ve = Veronica persica
Vi = Viola sp.

| Erfindungsgemäße Verbindung | Ze | Be | Br | Gl | Go | He | Se | Ab | Ga | Ip | Ma | Po | Sb | So | Ve | Vi |
|---|---|---|---|---|---|---|---|---|---|---|---|---|---|---|---|---|
| Beispiel 34 | 0 | 3 | 4 | 3 | 3 | 1 | 3 | 4 | 3 | 4 | 2 | 4 | 4 | 4 | 2 | 3 |
| Beispiel 36 | 0 | 4 | 3 | 2 | 4 | 4 | 3 | 4 | 3 | 4 | 4 | - | - | - | 4 | 3 |
| Beispiel 37 | 1 | 3 | 1 | 1 | 4 | 2 | 2 | 4 | 2 | 3 | 1 | - | - | - | 4 | 3 |
| Beispiel 52 | 0 | 4 | 1 | 0 | 4 | 3 | - | 4 | 3 | 4 | 2 | 1 | 4 | 4 | 4 | 2 |
| Unbehandelt | 0 | 0 | 0 | 0 | 0 | 0 | 0 | 0 | 0 | 0 | 0 | 0 | 0 | 0 | 0 | 0 |
| Vergleichsmittel | | | | | | | | | | | | | | | | |
| Oxadiazon | 2 | 4 | 4 | 2 | 4 | 2 | 3 | 4 | 4 | 4 | 3 | 4 | 4 | 4 | 4 | 4 |

Beispiel D

Im Gewächshaus wurden die aufgeführten Pflanzenspezies nach dem Auflaufen mit den aufgeführten Verbindungen in einer Aufwandmenge von 0,3 kg Wirkstoff/ha behandelt. Die Verbindungen wurden zu diesem Zweck als Emulsion mit 500 Litern Wasser/ha gleichmäßig über die Pflanzen versprüht. Hier zeigten zwei Wochen nach der Behandlung die erfindungsgemäßen Verbindungen eine hohe Kulturpflanzenselektivität in Soja bei ausgezeichneter Wirkung gegen das Unkraut. Das Vergleichsmittel zeigte nicht die gleichhohe Selektivität.

In der folgenden Tabelle bedeuten:
0 = keine Schädigung
1 = 1 - 24 % Schädigung
2 = 25 - 74 % Schädigung
3 = 75 - 89 % Schädigung
4 = 90 - 100 % Schädigung

Gl = Glycine maxima
Be = Beta vulgaris altissima
Br = Brassica sp.
Go = Gossypium hirsutum
He = Helianthus annuus
Ab = Abutilon theophrasti
Ga = Galium aparine
Ip = Ipomoea purpurea
Ma = Matricaria chamomilla
Po = Polygonum sp.
Se = Sesbania exaltata
So = Solanum sp.
Ve = Veronica persica
Vi = Viola sp.

| Erfindungsgemäße Verbindung | Gl | Be | Br | Go | He | Ab | Ga | Ip | Ma | Po | Se | So | Ve | Vi |
|---|---|---|---|---|---|---|---|---|---|---|---|---|---|---|
| Beispiel 46 | 1 | 4 | 4 | 4 | 2 | 4 | 3 | 4 | 2 | 2 | 3 | 4 | 4 | 4 |
| Beispiel 48 | 0 | 2 | 3 | 4 | 4 | 4 | 4 | 4 | 2 | 3 | 3 | 4 | 3 | 4 |
| Beispiel 50 | 1 | 2 | 3 | 4 | 1 | 4 | 4 | 3 | 2 | 3 | 2 | 4 | 3 | 4 |
| Beispiel 52 | 0 | 4 | 1 | 4 | 3 | 4 | 3 | 4 | 2 | 1 | 4 | 4 | 4 | 2 |
| Unbehandelt | 0 | 0 | 0 | 0 | 0 | 0 | 0 | 0 | 0 | 0 | 0 | 0 | 0 | 0 |
| Vergleichsmittel | | | | | | | | | | | | | | |
| Oxadiazon | 2 | 4 | 4 | 4 | 2 | 4 | 4 | 4 | 3 | 4 | 4 | 4 | 4 | 4 |

## Beispiel E

Im Gewächshaus wurden die in der Tabelle aufgeführten Verbindungen mit den ebenfalls erwähnten Aufwandmengen appliziert. Hierzu wurden die Wirkstoffe in Gefäße mit 1500 ml Wasser gegeben. Als Testpflanzenarten wurden Oryza sativa (Or), Echinochloa crus-galli (Ec), Cyperus difformis (Cy) und Eleocharis acicularis (El) im 2 - 5-Blatt-Stadium eingesetzt. Drei Wochen nach der Behandlung zeigte sich, daß die erfindungsgemäßen Verbindungen stark wirksam gegen wichtige Reisunkräuter und gleichzeitig selektiv zu Wasserreis sind.

In der folgenden Tabelle bedeuten:

0 = keine Schädigung
1 = schwache Schädigung
2 = mittlere Schädigung
3 = starke Schädigung
4 = total vernichtet

| Erfindungsgemäße Verbindung | Wasserapplikation ppm | Or | Ec | Cv | El |
|---|---|---|---|---|---|
| Beispiel 15 | 10 | - | 4 | 3 | 4 |
| Beispiel 35 | 1 | 0 | 4 | - | - |

Eine gleiche herbizide Wirkung zeigten auch die erfindungsgemäßen Verbindungen der Beispiele 1 bis 14,.16 bis 34 und 36 bis 53.

**Ansprüche**

1. Anellierte Imino-azole und Imino-azine der allgemeinen Formel I

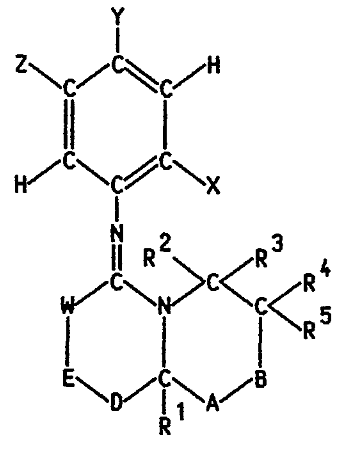

(I) ,

in der
A die Gruppe $(CR^6R^7)_n$,
B ein Sauerstoffatom oder die Gruppe $CR^8R^9$,
D die Gruppe $(CR^{10}R^{11})_n$,
E die Gruppe $CR^{12}R^{13}$,
W ein Sauerstoffatom oder die Gruppe $S(O)_m$,
X und Y unabhängig voneinander ein Wasserstoff- oder Halogenatom,
Z ein Wasserstoff- oder Halogenatom, einen Trihalogenmethylrest oder eine der Gruppen $-OR^{14}$, $-SR^{14}$,

$-NR_2^{14}$ oder $-CO_2R^{15}$,
$R^1$ ein Wasserstoffatom, einen $C_1$-$C_2$-Alkyl- oder Halogen-$C_1$-$C_2$-alkylrest,
$R^2$, $R^3$, $R^4$, $R^5$, $R^6$, $R^7$, $R^8$, $R^9$, $R^{10}$, $R^{11}$, $R^{12}$ und $R^{13}$ unabhängig vonein ander ein Wasserstoffatom, einen $C_1$-$C_4$-Alkylrest, einen Halogen-$C_1$-$C_4$-alkylrest, einen $C_1$-$C_4$-Alkoxyrest, einen $C_1$-$C_4$-Alkylthiorest, einen Halogen-$C_1$-$C_4$-alkoxyrest oder einen Halogen-$C_1$-$C_4$-alkylthiorest oder
$R^2$, $R^3$, $R^4$, $R^5$, $R^6$, $R^7$, $R^8$, $R^9$, $R^{10}$, $R^{11}$, $R^{12}$ und $R^{13}$ gegebenenfalls zusammen ein oder mehrere Ringe oder Alkylidengruppen,
$R^{14}$ ein Wasserstoffatom, einen gegebenenfalls durch ein oder mehrere Halogenatome substituierten $C_1$-$C_{12}$-Alkyl-, $C_3$-$C_8$-Cycloalkyl-, $C_3$-$C_6$-Alkenyl- oder $C_3$-$C_8$-Alkinylrest, einen Hydroxy-carbonyl-$C_1$-$C_8$-alkylrest, einen $C_1$-$C_4$-Alkoxy-, $C_2$-$C_4$-Alkenyloxy- oder $C_3$-$C_4$-Alkinyloxycarbonyl-$C_1$-$C_8$-alkylrest, einen $C_1$-$C_8$-Alkoxy-$C_1$-$C_8$-alkylrest, einen gegebenenfalls durch ein oder mehrere Halogenatome substituierten $C_1$- $C_4$-Alkylsulfonylrest, einen gegebenenfalls ein oder mehrfach, gleich oder verschieden durch Halogen, $C_1$-$C_4$-Alkyl, Halogen-$C_1$-$C_4$-alkyl, Nitro, $C_1$-$C_4$-Alkoxy oder Halogen-$C_1$-$C_4$-Alkoxy substituierten Phenylsulfonylrest, einen durch einen gesättigten oder ungesättigten Heterocyclus substituierten $C_1$-$C_4$-Alkylrest oder einen gegebenenfalls ein- oder mehrfach, gleich oder verschieden durch Halogen, $C_1$-$C_4$-Alkyl, Halogen-$C_1$-$C_4$-Alkyl, Nitro, $C_1$-$C_4$-Alkoxy oder Halogen-$C_1$-$C_4$-Alkoxy substituierten Phenyl- oder Phenyl-$C_1$-$C_4$-alkylrest,
$R^{15}$ ein Wasserstoffatom, einen gegebenenfalls durch ein oder mehrere Halogenatome substituierten $C_1$-$C_6$-Alkyl-, $C_2$-$C_6$-Alkenyl- oder $C_3$-$C_6$-Alkinylrest,
n 0 oder 1 und
m 0, 1 oder 2
bedeuten, und deren Salze mit anorganischen und organischen Säuren.

2. Verfahren zur Herstellung von anellierten Imino-azolen und Imino-azinen der allgemeinen Formel I gemäß Anspruch 1, dadurch gekennnzeichnet, daß man

A) eine Verbindung der allgemeinen Formel II

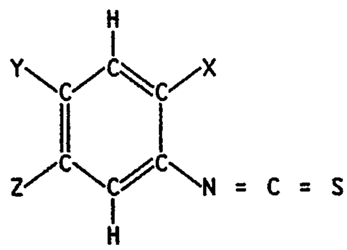

(II) ,

in der X, Y und Z die unter der allgemeinen Formel I angegebenen Bedeutungen haben, in einem Eintopfverfahren mit einer Verbindung der allgemeinen Formel III

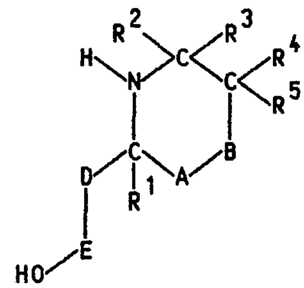

(III) ,

in der A, B, D, E, $R^1$, $R^2$, $R^3$, $R^4$ und $R^5$ die unter der allgemeinen Formel I angegebenen Bedeutungen haben, unter sauren Bedingungen cyclisiert,

B) falls in der allgemeinen Formel I W für ein Sauerstoffatom steht, eine Verbindung der allgemeinen Formel II

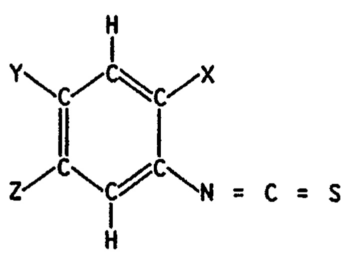

(II) ,

in der X, Y und Z die unter der allgemeinen Formel I angegebenen Bedeutungen haben, in einem Eintopfverfahren mit einer Verbindung der allgemeinen Formel III

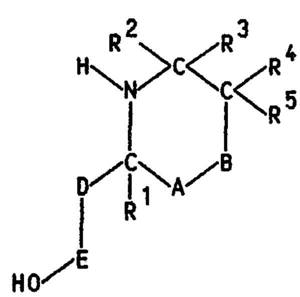

(III) ,

in der A, B, D, E, $R^1$, $R^2$, $R^3$, $R^4$ und $R^5$ die unter der allgemeinen Formel I angegebenen Bedeutungen haben, in einem geeigneten Lösungsmittel umsetzt,

C) einen Thioharnstoff der allgemeinem Formel IV

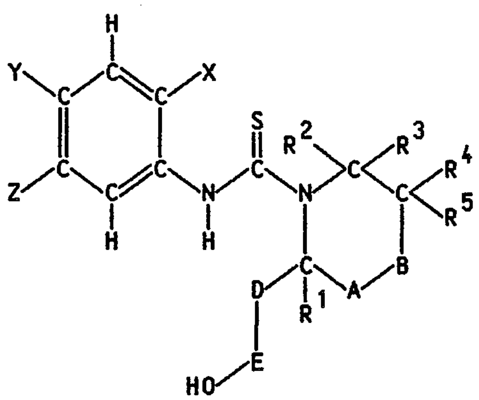

(IV) ,

in der A, B, D, E, X, Y, Z, $R^1$, $R^2$, $R^3$, $R^4$ und $R^5$ die unter der allgemeinen Formel I angegebenen Bedeutungen haben, unter sauren Bedingungen oder gegebenenfalls in einem geeigneten Lösungsmittel unter neutralen Bedingungen cyclisiert,

D) eine Verbindung der allgemeinen Formel V

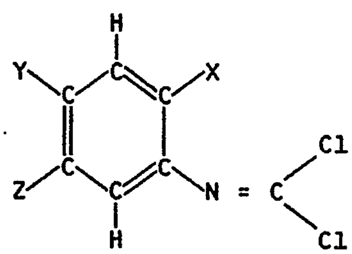

(V) ,

in der X, Y und Z die unter der allgemeine Formel I angegebenen Bedeutungen haben, mit einer Verbindung der allgemeinen Formel VI

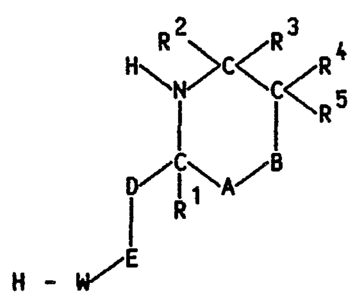

(VI) ,

in der A, B, D, E, W, $R^1$, $R^2$, $R^3$, $R^4$ und $R^5$ die unter der allgemeinen Formel I angegebenen Bedeutungen haben, in einem geeigneten Lösungsmittel unter basischen Bedingungen umsetzt,

E) falls in der allgemeinen Formel I Z für eine der Gruppen $-OR^{14}$, $-SR^{14}$ oder $-NR_2^{14}$ steht, $R^{14}$ aber nicht ein Wasserstoffatom, einen Phenylrest oder einen substituierten Phenylrest darstellt, eine Verbindung der allgemeinen Formel VII

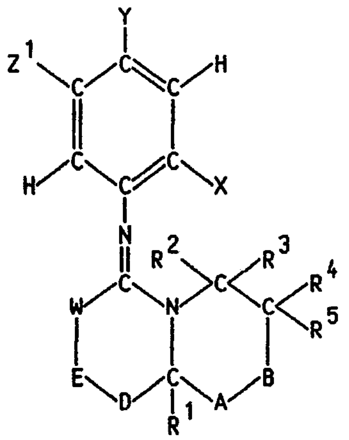

(VII) ,

in der A, B, D, E, W, X, Y, $R^1$, $R^2$, $R^3$, $R^4$ und $R^5$ die unter der allgemeinen Formel I angegebenen Bedeutungen haben und $Z^1$ für die Gruppen -OH, -SH oder $-NH_2$ steht, mit Verbindungen der allgemeinen Formel VIII

$R^{14}$ - G (VIII),

in der G für ein Halogenatom, einen p-Toluolsulfonyloxy- oder Methansulfonyloxyrest oder die Gruppe $-O-SO_2-OR^{14}$ steht und $R^{14}$ die unter der allgemeinen Formel I angegebene Bedeutung hat, aber nicht ein Wasserstoffatom, einen Phenylrest oder einen substituierten Phenylrest darstellt, gegebenenfalls unter Zusatz einer Base umsetzt,

F) falls in der allgemeinen Formel I Z für die Gruppe -OH steht, eine Verbindung der allgemeinen Formel IX

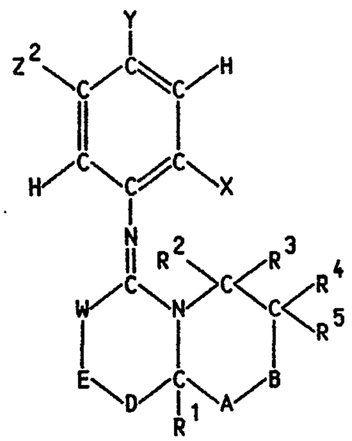

(IX) ,

in der A, B, D, E, W, X, Y, $R^1$, $R^2$, $R^3$, $R^4$ und $R^5$ die unter der allgemeinen Formel I angegebenen Bedeutungen haben und $Z^2$ für einen $C_1$-$C_{14}$-Alkyloxyrest oder einen $C_3$-$C_8$-Cycloalkyloxyrest steht, mit einer starken Mineralsäure einer Etherspaltung unterzieht oder

G) falls in der allgemeinen Formel I Z für eine der Gruppen -OH, -SH oder $-NH_2$ steht, eine Verbindung der allgemeinen Formel X

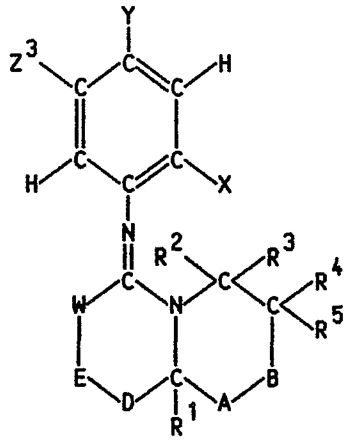

(X) ,

in der A, B, D, E, W, X, Y, $R^1$, $R^2$, $R^3$, $R^4$ und $R^5$ die unter der allgemeinen Formel I angegebenen Bedeutungen hat und $Z^3$ für eine der Gruppen -OR, -SR oder $NR_2$ mit R in der Bedeutung eines gegebenenfalls durch ein oder mehrere Halogenatome substituierten $C_1$-$C_4$-Alkylsulfonylrestes oder eines gegebenenfalls durch $C_1$-$C_3$-Alkyl oder Halogen substituierten Phenylsulfonylrestes steht, basisch hydrolysiert.

3. Mittel mit herbizider Wirkung, gekennzeichnet durch einen Gehalt an mindestens einer Verbindung gemäß dem Anspruch 1.

4. Verwendung von Mitteln gemäß dem Anspruch 3 zur Bekämpfung monokotyler und dikotyler Unkrautarten in landwirtschaftlichen Hauptkulturen.

5. Verfahren zur Herstellung von Mitteln mit herbizider Wirkung, dadurch gekennzeichnet, daß man Verbindungen der allgemeinen Formel I gemäß dem Anspruch 1 mit Träger- und/oder Hilfsstoffen vermischt.